Europäisches Patentamt

European Patent Office

Office européen des brevets

(19)

(11) Veröffentlichungsnummer : **0 116 340**
**B1**

(12) **EUROPÄISCHE PATENTSCHRIFT**

(45) Veröffentlichungstag der Patentschrift :
06.05.87

(21) Anmeldenummer : 84101040.8

(22) Anmeldetag : 02.02.84

(51) Int. Cl.⁴ : **C 07 C 11/02**, C 07 C   1/20,
**B 01 J 29/04**

(54) **Verfahren zur Herstellung von C2- bis C4-Olefinen aus Methanol/Dimethylether.**

(30) Priorität : **10.02.83 DE 3304479**

(43) Veröffentlichungstag der Anmeldung :
**22.08.84 Patentblatt 84/34**

(45) Bekanntmachung des Hinweises auf die Patenterteilung : **06.05.87 Patentblatt 87/19**

(84) Benannte Vertragsstaaten :
**BE DE FR GB NL**

(56) Entgegenhaltungen :
EP-A- 0 011 900
EP-A- 0 022 640
EP-A- 0 072 920
EP-A- 0 110 255

(73) Patentinhaber : **BASF Aktiengesellschaft**
**Carl-Bosch-Strasse 38**
**D-6700 Ludwigshafen (DE)**

(72) Erfinder : **Hoelderich, Wolfgang, Dr.**
**Mannheimer Strasse 18 c**
**D-6710 Frankenthal (DE)**
Erfinder : **Mross, Wolf Dieter, Dr.**
**Anselm-Feuerbach-Strasse 21**
**D-6710 Frankenthal (DE)**
Erfinder : **Schwarzmann, Matthias, Dr.**
**Carl-Bosch-Strasse 54**
**D-6703 Limburgerhof (DE)**

EP 0 116 340 B1

**Beschreibung**

In neuerer Zeit gewinnen Bemühungen, Methanol zur Herstellung von Olefinen zu verwenden, zunehmendes Interesse. Methanol läßt sich aus Kohle, über Kohlevergasung und Herstellung von Synthesegas, mit Hilfe bewährter Technologien leicht herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff beibehalten werden. In den vergangenen Jahren sind daher Verfahren entwickelt worden, die die Herstellung von Olefinen aus Methanol und/oder Dimethylether zum Gegenstand haben.

Ein solches Verfahren ist beispielsweise in der DE-OS 26 15 150 beschrieben. Als Katalysator wird ein Aluminosilikatzeolith ZSM-5 verwendet, der eigentlich ein Aromatisierungskatalysator ist. Die Umsetzung kann aber durch verschiedene Maßnahmen, insbesondere durch die Verkürzung der Verweilzeit, in Richtung Olefinbildung gelenkt werden. Weitere die Olefinbildung begünstigende Parameter sind insbesondere die Verdünnung von Methanol bzw. Dimethylether mit Inertgasen bzw. Wasserdampf. Die Erfahrung zeigt, daß hohe Olefinausbeuten nur durch eine sehr starke Verdünnung von Methanol und/oder Dimethylether mit Inertgas oder Wasserdampf zu erzielen sind. Andere bekannte Verfahren haben als Nachteil eine geringe Belastbarkeit und eine schnelle Verkokung des Katalysators. Die Verdünnung des Katalysators mit Bindemittel soll ebenfalls eine für die Olefinbildung vorteilhafte Maßnahme sein, doch werden durch die verwendeten Binder Nebenreaktionen und auch Desaktivierung des Katalysators verursacht.

Aus EP-A-22 640 war ein Verfahren zur Herstellung von Äthylen und höheren Kohlenwasserstoffen durch Abpaltung von Wasser aus Äthanol bekannt. Dabei entsteht allein Äthylen und daneben größere Mengen von höheren Kohlenwasserstoffen, deren Bildung im vorliegenden Falle verhindert werden soll. In den älteren Anmeldungen EP-A-72 920 und EP-A-110 255 wird jeweils Böhmit als Bindemittel vor der Verformung zugegeben.

Es wurde nun gefunden, daß man $C_2$- bis $C_4$-Olefine in hoher Ausbeute aus Methanol und/oder Dimethylether durch katalytische Umsetzung bei erhöhter Temperatur in Gegenwart von Borosilikatkatalysatoren erhält, wenn man als Katalysatoren Borosilikatzeolithe verwendet, die ohne Bindemittel tablettiert oder verstrangt und mit Fluorwasserstoff und anschließend mit Salzsäure behandelt wurden.

Vorteilhaft wendet man Katalysatoren an, die man mit Flußsäure, bevorzugt 0,1n HF, und anschließend mit Salzsäure, vorzugsweise mit 15 %iger HCl, behandelt, gründlich auswäscht, bei 100 °C trocknet und bei 500 °C calciniert. Erfindungswesentlich ist, daß beide nacheinander durchgeführten Maßnahmen (Flußsäure- und Salzsäurebehandlung) sich vorteilhafter auf die katalytischen Eigenschaften des Katalysators auswirken als die Einzelmaßnahmen.

Ein bevorzugtes Verfahren zur Herstellung der erfindungsgemäß verwendeten Katalysatoren besteht darin, die nach der Synthese in der Ammonium-Form vorliegenden Borosilikatzeolithe durch Calcination bei 540 °C/16 h in die acide H-Form überzuführen, und dieses calcinierte Produkt 1 bis 3 Stunden zwischen 60° und 80 °C mit einer 0,001n-1n Flußsäure, bevorzugt 0,05-0,2n HF, zu behandeln. Nach Abfiltrieren und Auswaschen wird der behandelte Zeolith bei 100 °C bis 140 °C getrocknet und bei 500 °C bis 600 °C/5 h calciniert. Dieser maßnahme schließt sich die Salzsäurebehandlung an, die zweckmäßig mit 3 bis 25 %iger, insbesondere mit 10-18 %iger Salzsäure zwischen 60 und 80 °C während 1 bis 3 Stunden durchgeführt wird. Das hierbei erhaltene Produkt wird abfiltriert, ausgewaschen, bis keine C-Ionen mehr im Waschwasser nachweisbar sind, getrocknet bei 100 °C bis 140 °C und bei 500 °C bis 600 °C/5 h calciniert.

Die Säurebehandlungen sind wesentlich für die Reaktionsfähigkeit des Katalysators ; der reine unbehandelte Borosilikatzeolith reagiert erst bei Temperaturen oberhalb 550 °C bzw. wenn Olefine zu- oder zurückgeführt werden bzw. reiner Dimethylether eingesetzt wird. Die hierbei erzielten Olefinausbeuten sind jedoch schlechter als bei den Katalysatoren mit Bindemittel unter gleichen Bedingungen. Die Säurebehandlungen ermöglichen die Chemiesorption des Methanols am reinen Borosilikatzeolith und die Dehydratation zu Dimethylether, dessen Bildung eine Voraussetzung für die Reaktion ist.

Bei der Durchführung des Verfahrens wird Methanol, das mit Dimethylether im Gleichgewicht steht, bei einem Druck zwischen Normaldruck und etwa 30 bar, vorzugsweise bei 0 bis 1 bar und bei Temperaturen zwischen 300 °C und 650 °C, vorzugsweise bei 400 °C bis 550 °C, an den oben beschriebenen Katalysatoren umgesetzt. Das Methanol kann einen Wassergehalt bis zu 90 Gew.-% haben, zweckmäßig verwendet man als Ausgangsstoff Rohmethanol, das etwa 20 % Wasser enthält.

Dem Methanol können auch noch andere niedere Alkohole beigemischt sein. Die Belastung des Katalysators, ausgedrückt in WHSV = $h^{-1}$ — g Methanol und/oder Dimethylether pro g Katalysator und Stunde —. wird zweckmäßig so gewählt, daß die Ausgangsstoffe möglichst quantitativ umgesetzt werden, so daß keine Abtrenn- und Rückführprobleme für nicht umgesetzten Dimethylether entstehen. Im allgemeinen soll daher die WHSV im Bereich von 0,5 bis 50 $h^{-1}$, vorzugsweise im Bereich von 2 bis 15 $h^{-1}$ liegen.

Durch das erfindungsgemäße Verfahren wird die Olefinselektivität im $C_2$-bis $C_4$-Bereich bei der Methanolumwandlung zu Kohlenwasserstoffen, insbesondere im Temperaturbereich von 400 °C bis 600 °C wesentlich erhöht.

2

0 116 340

Die unerwünschten Nebenprodukte Methan und Aromaten, deren Bildung teilweise vom Bindemittel verursacht werden, werden sich durch Verwendung des erfindungsgemäßen Katalysators stark zurückgegrängt. Diese Tatsache äußert sich vorteilhaft ; es wird eine Erhöhung der Laufzeit des eingesetzten Katalysators bewirkt.

Unter Laufzeit versteht man die Zeit zwischen Regenerierungen. Auch die Lebensdauer des Katalysators wird insgesamt verlängert. Der erfindungsgemäße Effekt der Laufzeitverbesserung wirkt sich besonders bei der Umsetzung bei hohen Temperaturen, z. B. im Bereich von 450 °C bis 550 °C, aus.

Auch ist es von wirtschaftlicher Bedeutung, daß eine Verstrangung oder Tablettierung des Borosilikatzeolithen mit Bindemittel unterbleibt. Es ist ein weiterer Vorteil der Erfindung, daß man die Umsetzung zu $C_2$- bis $C_4$-Olefinen mit Rohmethanol ohne weiteren Zusatz von inerten Verdünnungsmittel wie $N_2$, He oder $H_2O$ ausführen kann.

Die Durchführung des erfindungsgemäßen Verfahrens wird anhand der nachstehenden Beispiele näher erläutert.

Beispiele

Der Bor-Zeolith wird in einer hydrothermalen Synthese aus hochdispersen 64 g $SiO_2$ 12,2 g $H_3BO_3$, 800 g einer wäßrigen 1,6-Hexandiamin-Lösung (Mischung 50 : 50) bei 170 °C unter autogenem Druck in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Borosilikatzeolith setzt sich zusammen aus 94,2 Gew.-% $SiO_2$ und 2,32 Gew.-% $B_2O_3$.

Katalysator A

50 g dieses Borosilikatzeolithen werden mit 140 ml 0,1n HF unter Rückfluß 1 h behandelt. Nach der Filtration und Auswaschen mit Wasser wird bei 110 °C/16 h getrocknet und bei 500 °C/5 h calciniert, danach zu 3 mm-Pillen tablettiert oder zu 2 mm-Strängen verpreßt.

Katalysator B

50 g Borosilikatzeolith — in tablettierter oder verstrangter Form — werden mit 250 ml 18 %iger Salzsäure 1 h bei 80 °C behandelt, danach abfiltriert und mit Wasser chloridfrei gewaschen. Dieses Produkt wird bei 110 °C 16 h getrocknet und bei 500 °C 5 h calciniert.

Katalysator C (erfindungsgemäß)

Bei Katalysator C werden die beim Katalysator A und B beschriebenen Maßnahmen kombiniert, d. h. der Borosilikatzeolith wird einer Flußsäure- und anschließenden Salzsäurebehandlung unterworfen.

An diesen Katalysatoren A, B, C werden unter isothermen Bedingungen in einem Rohrreaktor Rohmethanol mit 20 Gew.-% Wasser bei 550 °C und WHSV = 7,8 $h^{-1}$ bez. auf eingesetztes $CH_3OH$, quantitativ umgesetzt. Die Ausbeuten, bezogen auf eingesetztes $CH_2$, sind in der Tabelle, Spalte A, B, C angegeben.

Zum Ausbeutevergleich wurde noch der folgende Katalysator D herangezogen, der unter denselben Reaktionsbedingungen wie die Katalysatoren A, B, C getestet wurde.

Katalysator D

wird erhalten durch Verstrangen des oben beschriebenen Borosilikatzeolithen mit Böhmit im Verhältnis 60 : 40. Die Trocknung erfolgt bei 110 °C h und die Calcinierung bei 500 °C/16 h.

| Katalysator | A | B | C | D |
|---|---|---|---|---|
| $C_2H_4$ | 12,1 | 6,7 | 11,9 | 12,0 |
| $C_3H_6$ | 40,8 | 42,9 | 43,4 | 32,9 |
| $C_4H_8$ | 21,1 | 20,9 | 22,8 | 16,1 |
| $CH_4$ | 1,4 | 1,2 | 1,5 | 7,9 |
| $C_2H_6$ | 0,3 | | 0,3 | 0,6 |
| $C_3H_8$ | 1,6 | 0,6 | 1,4 | 2,1 |
| $C_4H_{10}$ | 1,0 | 1,0 | 0,9 | 1,2 |
| $C_5$-Aliphate | 10,7 | 20,3 | 12,3 | 9,4 |
| $C_6$-Aromaten | 9,0 | 5,6 | 5,6 | 15,9 |
| Laufzeit h | 14 | 12 | 90 | 7 |
| g $CH_3OH$/g Katalysator | 109 | 94 | 702 | 55 |

3

**0 116 340**

Patentansprüche

1. Verfahren zur Herstellung von $C_2$- bis $C_4$-Olefinen durch katalytische Umsetzung von Methanol und/oder Dimethylether in Gegenwart von Borosilikatzeolithen bei erhöhter Temperatur, dadurch gekennzeichnet, daß man Borosilikatzeolithe verwendet, die ohne Bindemittel tablettiert oder verstrangt und mit Fluorwasserstoff und anschließend mit Salzsäure, behandelt wurden.

2. Verfahren nach Anspruch 1, dadurch gekennzeichnet, daß man für die Fluorwasserstoffbehandlung der Katalysatoren 0,001n-1n HF und für die anschließende Salzsäurebehandlung 3 bis 25 %ige Säure einsetzt.

**Claims**

1. A process for the preparation of $C_2$-$C_4$-olefins by catalytic conversion of methanol and/or dimethyl ether in the presence of a borosilicate zeolite at elevated temperature, wherein a borosilicate zeolite is used which has been tabletted or extruded without a binder, and treated with hydrofluoric acid and then with hydrochloric acid.

2. A process as claimed in claim 1, wherein 0.001-1N HF is used for the hydrofluoric acid treatment of the catalyst, and 3 to 25 % strength acid is used for the subsequent hydrochloric acid treatment.

**Revendications**

1. Procédé de préparation d'oléfines en $C_2$ à $C_4$ par transformation catalytique de méthanol et/ou de diméthyléther en présence de zéolites de borosilicate à température élevée, caractérisé en ce qu'on utilise des zéolites de borosilicate qui ont été mises sous forme de bâtonnets ou de comprimés sans liant et qui ont été traitées par l'acide fluorhydrique, puis par l'acide chlorhydrique.

2. Procédé selon la revendication 1, caractérisé en ce qu'on utilise HF 0,001N à 1N pour le traitement des catalyseurs par l'acide fluorhydrique et un acide à 3-25 % pour leur traitement consécutif par l'acide chlorhydrique.